# EUROPÄISCHE PATENTSCHRIFT

(11) **EP 1 432 830 B1**
(45) Veröffentlichungstag und Bekanntmachung des Hinweises auf die Patenterteilung: **30.08.2017**
(21) Anmeldenummer: 02795056.7
(22) Anmeldetag: 26.09.2002
(51) Int. Cl.: C12Q 1/68, C12N 15/10

(54) **VERFAHREN ZUR ISOLIERUNG VON DNA AUS BIOLOGISCHEN PROBEN**
METHOD FOR ISOLATING DNA FROM BIOLOGICAL SAMPLES
PROCEDE POUR ISOLER DE L'ADN A PARTIR D'ECHANTILLONS BIOLOGIQUES

(30) Priorität: 26.09.2001 DE 10147439
(43) Veröffentlichungstag der Anmeldung: 30.06.2004
(73) Patentinhaber: QIAGEN GmbH, 40724 Hilden (DE)
(72) Erfinder: LENZ, Christian, 40233 Düsseldorf (DE)
(86) Internationale Anmeldenummer: PCT/EP2002/010823
(87) Internationale Veröffentlichungsnummer: WO 2003/035903

(56) Entgegenhaltungen:
- US-A- 6 130 073
- PARZER S ET AL: "A RAPID METHOD FOR THE ISOLATION OF GENOMIC DNA FROM CITRATED WHOLE BLOOD" BIOCHEMICAL JOURNAL, Bd. 273, Nr. 1, 1991, Seiten 229-232, XP009007536 ISSN: 0264-6021
- CIULLA T A ET AL: "A SIMPLE METHOD FOR DNA PURIFICATION FROM PERIPHERAL BLOOD" ANALYTICAL BIOCHEMISTRY, Bd. 174, Nr. 2, 1988, Seiten 485-488, XP009007530 ISSN: 0003-2697
- GRIMBERG ET AL: "A SIMPLE AND EFFICIENT NON-ORGANIC PROCEDURE FOR THE ISOLATION OF GENOMIC DNA FROM BLOOD" NUCLEIC ACIDS RESEARCH, OXFORD UNIVERSITY PRESS, SURREY, GB, Bd. 17, Nr. 20, 1989, Seite 8390 XP002100596 ISSN: 0305-1048
- LAHIRI DEBOMOY K ET AL: "A non-organic and non-enzymatic extraction method gives higher yields of genomic DNA from whole-blood samples than do nine other methods tested." JOURNAL OF BIOCHEMICAL AND BIOPHYSICAL METHODS, Bd. 25, Nr. 4, 1992, Seiten 193-205, XP009007519 ISSN: 0165-022X
- "innuPREP Plasmid Mini Kit Plus", , 19 June 2015 (2015-06-19), Retrieved from the Internet: URL:http:analytik-jena.de [retrieved on 2015-06-19]
- "peqGOLD Plant DNA Mini Kit", PEQLAB, 26 August 2015 (2015-08-26),

## Beschreibung

Die vorliegende Erfindung betrifft ein verbessertes Verfahren zur Isolierung von DNA aus biologischen Proben, insbesondere aus humanem Vollblut.

Die Reinigung von Nukleinsäuren spielt eine zentrale Rolle in der Molekularbiologie. Die aufgereinigte DNA dient dabei unter anderem als Ausgangsmaterial für die Nukleinsäurebasierende Diagnostik, für das Erstellen genetischer Profile und für die Pharmakogenomik. Daher kommt der Isolierung von Nukleinsäuren wie DNA und RNA aus biologischen Proben - wie zum Beispiel Blut, Körpersekreten, Gewebeproben, Urin, Stuhl und dergleichen - zum nachfolgenden Einsatz in genetischen Analysen eine besondere Bedeutung zu.

Bei den aus dem Stand der Technik bekannten Methoden zur Gewinnung von Nukleinsäuren erfolgt die Isolierung von DNA aus Zellen und Geweben dadurch, dass die Ausgangsmaterialien unter stark denaturierenden und reduzierenden Bedingungen, teilweise auch unter Verwendung von proteinabbauenden Enzymen, aufgeschlossen, die austretenden Nukleinsäurefraktionen über Phenol-/Chloroform-Extraktionsschritte gereinigt und die Nukleinsäuren mittels Dialyse oder Ethanolpräzipitation aus der wässrigen Phase gewonnen werden [Sambrook, J., Fritsch, E.F. in T. Maniatis, CSH, "Molecular Cloning", 1989].

Diese seit langem etablierten Verfahren zur Isolierung von DNA aus Zellen und insbesondere aus Geweben weisen jedoch gravierende Nachteile auf: zum einen sind sie sehr zeitintensiv und erfordern einen nicht unerheblichen experimentellen Aufwand. Daneben sind derartige Verfahrensweisen infolge der zu verwendenden Chemikalien - wie Chloroform oder Phenol - mit der akuten Gefahr einer Gesundheitsschädigung der mit der Isolierung befassten Personen verbunden.

Die zwangsläufige Folge der oben geschilderten Nachteile war, dass im Laufe der Zeit alternative Verfahren zur Isolierung von Nukleinsäuren aus unterschiedlichen biologischen Ausgangsmaterialien entwickelt wurden, mit denen die aufwendige und gesundheitsschädigende Phenol-/Chloroform-Extraktion von Nukleinsäuren vermieden sowie eine Reduzierung des Zeitaufwandes erreicht werden sollte.

Alle diese aus dem Stand der Technik bekannten Verfahrensvorschläge basieren auf einer von Vogelstein und Gillespie [Proc. Natl. Acad. Sci. USA, 1979, 76, 615-619] entwickelten und erstmals beschriebenen Methode zur präparativen und analytischen Reinigung von DNA-Fragmenten aus Agarosegelen. Die Methode kombiniert die Auflösung der DNA-Bande enthaltenden Agarose in einer chaotropen Salzlösung mit der Bindung der DNA an Trägerpartikel. Anschließend wird die fixierte DNA mit einer Waschlösung (20 mM EDTA; 50% v/v Ethanol) gewaschen und danach von den Trägerpartikeln abgelöst.

Diese Methode erfuhr bis heute eine Reihe von Modifikationen und wird bis zum gegenwärtigen Zeitpunkt für unterschiedliche Verfahren der Extraktion und Reinigung von Nukleinsäuren aus unterschiedlichen Quellen angewendet (Marko, M. A., Chipperfield, R. und Birnboim, H.G., 1982, Anal. Biochem. 121, 382-387).

Darüber hinaus existieren heute weltweit auch eine Vielzahl von Reagenziensystemen, vor allem zur Reinigung von DNA-Fragmenten aus Agarosegelen und für die Isolierung von Plasmid DNA aus bakteriellen Lysaten, aber auch für die Isolierung von längerkettigen Nukleinsäuren (genomische DNA, zelluläre Gesamt-RNS) aus Blut, Geweben oder Zellkulturen.

Viele dieser kommerziell verfügbaren Aufreinigungssysteme basieren auf dem hinlänglich bekannten Prinzip der Bindung von Nukleinsäuren an mineralische Träger unter Anwesenheit von Lösungen unterschiedlicher chaotroper Salze. In diesen Systemen werden als Trägermaterialien Suspensionen feingemahlener Glaspulver, Diatomeenerden oder auch Silicagele eingesetzt.

Ein für eine Vielzahl unterschiedlicher Anwendungen praktikables Verfahren zur Isolierung von Nukleinsäuren ist in der U.S.-Patentschrift 5 234 809 dargestellt. Dort wird ein Verfahren zur Isolierung von Nukleinsäuren aus nukleinsäurehaltigen Ausgangsmaterialien durch die Inkubation des Ausgangsmaterials mit einem chaotropen Puffer und einer DNA-bindenden festen Phase offenbart. Die chaotropen Puffer realisieren sowohl die Lyse des Ausgangsmaterials wie auch die Bindung der Nukleinsäuren an die feste Phase. Das Verfahren wird dazu benutzt, um Nukleinsäuren aus kleinen Probenmengen zu isolieren und findet speziell im Bereich der Isolierung viraler Nukleinsäuren seine praktische Anwendung.

Das physikochemische Prinzip der nach dem bekannten Stand der Technik heute eingesetzten und kommerziell verfügbaren Systeme zur Isolierung von Nukleinsäuren auf der Basis der Bindung von Nukleinsäuren an die Oberflächen mineralischer Träger soll dabei in der Störung übergeordneter Strukturen des wässerigen Milieus bestehen, durch welche die Nukleinsäuren an der Oberfläche von mineralischen Materialien, insbesondere von Glas- bzw. Silicapartikeln adsorbieren. Die Störung der übergeordneten Strukturen des wässerigen Milieus erfolgt dabei immer unter Anwesenheit chaotroper Ionen und ist bei hohen Konzentrationen dieser fast quantitativ. Auf dieser beschriebenen physiko-chemischen Basis enthalten viele kommerziell verfügbaren Systeme zur Isolierung von Nukleinsäuren Pufferkompositionen mit chaotropen Salzen, die hohe Ionenstärken aufweisen, welche für die Bindung von Nukleinsäuren an eine Nukleinsäuren-bindende feste Phase als erforderlich angesehen werden.

Gravierende Nachteile des Verfahrens bestehen aber u.a. darin, dass der durch die chaotropen Puffer realisierte Aufschluss längst nicht für alle Materialien einsetzbar ist bzw. auch für größere Mengen an Ausgangsmaterialien nur extrem ineffizient und unter einem großen Zeitaufwand realisiert werden kann. Darüber hinaus müssen für unterschiedliche Aufgabenstellungen auch immer verschieden hohe Konzentrationen unterschiedlicher chaotroper Puffer eingesetzt werden, was sich als sehr aufwendig erweist.

Aus dem Stand der Technik ist auf der anderen Seite die Isolierung von Nukleinsäuren auf der Basis eines Aussalzungsschritts bekannt [L.A. Salazar et al., Clin. Chem. 44 (1998) 1748; S.A. Miller et al., Nucleic Acid Res., 16 (3) (1988) 1215]. Diese Verfahren beinhalten jedoch den Nachteil, dass während der Verfahrensdurchführung mindestens einmal der Wechsel des Reaktionsgefäßes erforderlich wird, was die Gefahr der Probenverwechslung in sich birgt. Darüber hinaus lassen sich die ausgesalzten Proteine nur durch die experimentell aufwendige Methode der Zentrifugation von der zu reinigenden Nukleinsäure abtrennen.

Ein weiteres Verfahren zur Reinigung von DNA, die aus Blut gewonnen wurde, wird von T.A. Ciulla offenbart [Analytical Biochemistry, 174 (1988) 485]. Hierbei werden zunächst die Zellkerne durch einen Lyseschritt freigesetzt. Die darin enthaltene DNA wird unter Verwendung von Guanidinium-*iso*-thiocyanant und β-Mercaptoethanol und durch anschließende Fällung mit 2-Propanol isoliert. Dieses Verfahren beinhaltet jedoch relativ zeitaufwendige Resuspendier- und Zentrifugationsschritte. Außerdem verwendet dieses Verfahren einen Lysepuffer, der anfällig gegenüber mikrobiellem Bewuchs ist und im Verhältnis zum Blut einen 9-fachen Überschuss erfordert. Darüber hinaus wird *β-*Mercaptoethanol als giftige Substanz eingestuft.

Parzer et al. (Biochem. J. (1991) 273, 229-231) beschreibt ein vergleichsweise schnelles Verfahren zur Isolierung von DNA mittels nicht-chaotroper Lysebedingungen, das zwar auf die Verwendung toxischer Reagenzien verzichten kann, aber nicht bis zum Erhalt der isolierten DNA in demselben Gefäß durchgeführt werden kann.

In der Literatur sind diverse Verfahren zur Isolierung von Nukleinsäuren mittels mehrstufigen Lyse- bzw. Suspendierungsschritten beschrieben, die sich insbesondere in der Zusammensetzung ihrer Lyse- und Suspendierungslösungen unterscheiden.

So beschreibt US 6,130,073 ein PCR-basiertes Verfahren zur Analyse komplexer genetischer Systeme. Die dafür benötigte DNA wird aus mit EDTA oder Citrat stabilisiertem Vollblut in einem mehrschrittigen Verfahren erhalten, das zunächst einen Lyseschritt unter Verwendung von Triton X-100 und MgCl₂ aufweist und anschließend das erhaltene Pellet unter chaotropen Bedingungen resuspendiert.

Gemäß Davidson (Ann Clin Biochem (2002), 39, 273-280, in Kombination mit Am J. Clin Pathol (1993), 100, 371-2) liegen optimale EDTA Konzentrationen in stabilisiertem Blut dabei bei 4-55 mmol pro Liter Blut.

Grimberg et al. (Nucleic Acid Research (1989) Vol. 17, No. 20, S. 8390) beschreibt zur Isolierung der Nukleinsäure ein ähnliches Verfahren, bei dem das nach der Lyse erhaltene Pellet allerdings unter nicht-chaotropen Bedingungen in einem Proteinase K haltigen Puffer suspendiert wird.

Lahiri et al. (Journal of Biochemical and Biophysical Methods, 25 (1992), 193-205) verwendet sowohl zur Lyse als auch zum Resuspendieren eine Kombination aus EDTA und mehreren Salzen, bei der Lyse zusätzlich noch ein nichtionisches und bei der Resuspension zusätzlich noch ein anionisches Detergenz. Das Verfahren kann allerdings nicht bis zum Erhalt der DNA in einem Reaktionsgefäß durchgeführt werden.

Die Aufgabe der vorliegenden Erfindung besteht somit darin, ein Verfahren zur Verfügung zu stellen, das die Nachteile der aus dem Stand der Technik bekannten Verfahren vermeidet.

Insbesondere besteht die Aufgabe der vorliegenden Erfindung darin, bei der Isolierung von DNA auf toxische oder ätzende Substanzen zu verzichten.
Daneben stellt sich die vorliegende Erfindung die Aufgabe, ein zeitsparendes und wenig aufwendiges Verfahren zur Isolierung von DNA vorzuschlagen.

Eine weitere Aufgabe der vorliegenden Erfindung ist es, ein Verfahren zu schaffen, das ohne Wechsel des Reaktionsgefäßes - als sog. "Eintopfreaktion" - durchführbar ist und damit die Verwechslungsgefahr bzw. eine Konatamination der Proben auf ein Minimum reduziert.

Die vorgenannten Aufgaben werden erfindungsgemäß dadurch gelöst, dass man die biologische Probe in einem Reaktionsgefäß mit einem Lysereagenz, das Salze einwertiger Kationen, Salze zweiwertiger Kationen, einen oder mehrere Komplexbildner ausgewählt aus Ethylendiamintetraacetat, Ethylendioxy-bis-(ethylennitrilo)tetraessigsäure, DTPA und Na₅P₃O₁₀ und Octylphenol polyethoxylat enthält, vermischt. Im nachfolgenden Schritt werden die DNA-enthaltenden Zellkomponenten von den übrigen Zellkomponenten getrennt. Diese Trennung kann zum Beispiel durch Zentrifugation erfolgen.

Die im Sediment enthaltene DNA wird nachfolgend von weiteren Verunreinigungen, zum Beispiel Proteinen, abgetrennt. Diese Trennung erreicht man durch Resuspendieren des Sediments in einer Salzlösung, wobei die Salzlösung mindestens ein chaotropes Salz enthält. Optional können die Verunreinigungen auch durch Erhitzen und / oder durch den Einsatz von Enzymen von der DNA getrennt werden. Die DNA wird anschließend durch Alkoholzugabe ausgefällt und das Präzipitat von der Lösung getrennt. Dies kann zum Beispiel durch Zentrifugation oder durch das Aufspulen der DNA auf einen Glashaken erfolgen. Nach Waschen mit einer Waschflüssigkeit, in der sich Salze, aber nicht die DNA lösen, und Trocknen wird die DNA in einem geeigneten Puffer resuspendiert. Die Isolierung wird in einem Reaktionsgefäß durchgeführt.

Als Nukleinsäure-enthaltende biologische Probe können zellfreies Probenmaterial, Plasma, Körperflüssigkeiten, wie beispielsweise Blut, Buffy Coat, Zellen, Leukozytenfraktionen, Crusta Phlogistica, Sputum, Urin, Sperma oder Organismen (Ein- oder Mehrzeller; Insekten etc.) bzw. Eukaryonten oder Prokaryonten verwendet werden.

Für die Durchführung des erfindungsgemäßen Verfahrens eignen sich insbesondere Blut (humanes Vollblut), Buffy Coat, Leukozytenfraktionen und Zellkulturen.

Ganz besonders eignet sich für die Durchführung des erfindungsgemäßen Verfahrens Blut (humanes Vollblut).

Als Lysereagenzien eignen sich zur Durchführung des erfindungsgemäßen Verfahrens die aus dem Stand der Technik bekannten Detergentien-haltigen Lysereagenzien. Geeignete Detergentien sind aus dem Stand der Technik in großer Zahl bekannt.

Zur Lösung der erfindungsgemäßen Aufgabe eignet sich insbesondere Triton X-100 (Octylphenol polyethoxylat).

Erfindungsgemäß werden bevorzugt Lysereagenzien auf der Basis von 0,5 bis 7,5 Vol.-% Detergenz eingesetzt, wobei ein Gehalt von 1,0 bis 5,0 Vol.-% besonders bevorzugt wird.

Dem Lysereagenz werden Salze ein- und zweiwertiger Kationen sowie Komplexbildner zugesetzt.

Erfindungsgemäß werden bevorzugt Lysereagenzien auf der Basis von 0,5 bis 200 mM Magnesiumchlorid, 0,5 bis 200 mM Kaliumchlorid sowie 0,5 bis 100 mM Ethylendiamintetraacetat und/oder Ethylendioxy-bis-(ethylennitrilo)-tetra-essigsäure eingesetzt.

Besonders bevorzugt werden Lysereagenzien auf der Basis von 1 bis 30 mM Magnesiumchlorid, 1 bis 20 mM Kaliumchlorid sowie 1 bis 10 mM Ethylendiamintetraacetat und/oder 1 bis 10 mM Ethylendioxy-bi(ethylennitrilo)tetraessigsäure eingesetzt.

Gegebenenfalls können dem Lysereagenz eine oder mehrere Puffersubstanzen zugesetzt werden. Der pH-Wert des Lysereagenz kann über einen weiten Bereich variiert werden und liegt zur Durchführung des erfindungsgemäßen Verfahrens bevorzugt in einem Bereich von pH 2 bis 10 und besonders bevorzugt in einem Intervall von pH 3 bis 9. Dabei können die dem Fachmann bekannten Puffersysteme zum Einstellen des pH-Werts verwendet werden. Erfindungsgemäß werden bevorzugt auf Tris(hydroxymethyl)aminomethan basierende Puffersysteme verwendet. Besonders bevorzugt wird ein Puffersystem auf der Basis von 0,5 bis 250 mM Tris(hydroxymethyl)aminomethan Hydrochlorid.

Ganz besonders bevorzugt wird ein Lysepuffer eingesetzt, der 13 mM Tris(hydroxymethyl)aminomethan Hydrochlorid, pH 8,0, 10 bis 14,5 mM Magnesium(II)chlorid Hexahydrat, 10 mM Kaliumchlorid, 2 bis 5 mM Ethylendiamintetraacetat und/oder 1 bis 2 mM Ethylendioxy-bis(ethylennitrilo)tetraessigsäure sowie 2 Vol.-% Triton X-100 enthält.

Als Salzlösung zum Resuspendieren des Nukleinsäure-haltigen Sediments eignen sich zur Durchführung des erfindungsgemäßen Verfahrens die aus dem Stand der Technik einschlägig bekannten Lösungen auf der Basis hoher Salzkonzentrationen mit mindestens einem chaotropen Salz, insbesondere unter Verwendung von chaotropen Salzen. Unter chaotropen Salzen werden im Sinne der Erfindung Salze verstanden, die eine hohe Affinität zu Wasser haben und deshalb eine große Hydrathülle ausbilden; geeignete chaotrope Salze sind aus dem Stand der Technik in großer Zahl bekannt.

Erfindungsgemäß werden bevorzugt einzeln oder in Kombination Harnstoff-haltige, Guanidinium-*iso*-Thiocyanat-haltige oder Guanidinium-Hydrochlorid-haltige Lösungen eingesetzt, worunter letztere besonders bevorzugt werden.

Insbesondere werden bevorzugt Lösungen auf der Basis von 0,5 bis 7,0 M Guanidinium-Hydrochlorid zur Lösung der erfindungsgemäßen Aufgaben eingesetzt.

Besonders bevorzugt werden Lösungen auf der Basis von 2,0 bis 4,0 M Guanidinium-Hydrochlorid eingesetzt.

Derartige Denaturierungslösungen können ggf. weitere Substanzen - wie z.B. Komplexbildner oder Puffersubstanzen - enthalten. Der pH-Wert des Denaturierungspuffers kann zur Durchführung des erfindungsgemäßen Verfahrens über ein weites Intervall variieren; er liegt bevorzugt in einem pH-Bereich von 3 bis 10 und besonders bevorzugt in einem pH-Intervall von 7,5 bis 9,5. Dabei können die dem Fachmann bekannten Puffersysteme zum Einstellen des pH-Werts verwendet werden. Erfindungsgemäß werden bevorzugt auf Tris(hydroxymethyl)aminomethan basierende Puffersysteme verwendet. Besonders bevorzugt wird ein Puffersystem auf der Basis von 0,5 bis 250 mM Tris(hydroxymethyl)aminomethan Hydrochlorid.

Ganz besonders bevorzugt kommt ein Denaturierungspuffer mit 3 M Guanidinium-Hydrochlorid und 50 mM Tris(hydroxymethyl)aminomethan Hydrochlorid, pH 8,5, zur Verwendung.

Zur Unterstützung der denaturierenden Wirkung des Denaturierungspuffers kann die Probe ggf. erwärmt werden. Dabei liegt die Reaktionstemperatur dieses optionalen Schritts des erfindungsgemäßen Verfahrens in einem Intervall von 15 bis 95°C, bevorzugt in einem Intervall von 30 bis 85°C und besonders bevorzugt wird dieser Schritt bei einer Temperatur von 65°C durchgeführt.

Zur weiteren Abtrennung der DNA von Verunreinigungen kann die Probe ggf. mit Enzymen behandelt werden. Zur Durchführung des erfindungsgemäßen Verfahrens sind Proteasen, Lipasen, Amylasen und weitere dem Fachmann bekannte degradierende Enzyme oder deren Gemische geeignet.

Zur Ausfällung der DNA eignen sich zur Durchführung des erfindungsgemäßen Verfahrens die aus dem Stand der Technik einschlägig bekannten Alkohole wie verzweigte oder unverzweigte C₁-C₄-Alkanole. Besonders geeignet erweisen sich Propan-2-ol (*iso*-Propanol) und Ethanol.

Als Waschflüssigkeit wird insbesondere eine Mischung aus einem verzweigten oder unverzweigten C₁-C₄-Alkanol mit Wasser eingesetzt, worunter wässrige Lösungen von Ethanol besonders bevorzugt werden. Besonders bevorzugt wird eine 70 Vol.-prozentige Lösung von Ethanol in Wasser.

Als "Niedrigsalzlösungen" zum Resuspendieren der DNA Präzipitate eignen sich alle Flüssigkeiten, die DNA zu lösen vermögen. Lösungen oder geeignete Puffer sind aus dem Stand der Technik ebenfalls in großer Zahl bekannt.

Erfindungsgemäß werden Niedrigsalzlösungen (Lösungen mit geringer Ionenstärke) bevorzugt, worunter erfindungsgemäß auch Wasser fällt.

Derartige Lösungen können ggf. weitere Substanzen - wie z.B. Komplexbildner oder Puffersubstanzen - enthalten. Der pH-Wert des Puffers kann über einen weiten Bereich variieren und liegt bevorzugt in einem Bereich pH 6 bis 10 und besonders bevozugt in einem Intervall von pH 7,5 bis 9,5. Dabei können die dem Fachmann bekannten Puffersysteme zum Einstellen des pH-Wertes verwendet werden. Erfindungsgemäß werden bevorzugt auf Tris(hydroxymethyl)aminomethan basierende Puffersysteme verwendet. Besonders bevorzugt wird ein Puffersystem auf der Basis von 0,5 bis 150 mM Tris(hydroxymethyl)aminomethan Hydrochlorid.

Ganz besonders bevorzugt kommt als Hydrierungspuffer eine Lösung mit 10 mM Tris(hydroxymethyl)aminomethan Hydrochlorid, pH 8,5 zur Anwendung.

Erläuterung der Zeichnungen:
Fig. 1 zeigt die elektrophoretische Analyse einer gemäß Beispiel 1 aus humanem Vollblut isolierten DNA
Fig. 2 zeigt die elektrophoretische Analyse einer Polymerase-Kettenreaktion (PCR) mit dem Ziel der Amplifikation eines Einzelkopie-Gens (human analog of giant larvae gene)
Fig. 3 zeigt die elektrophoretische Analyse einer gemäß Beispiel 3 aus einer Zellkultur isolierten DNA

Die nachfolgenden Beispiele sollen die vorliegende Erfindung demonstrieren:

### Beispiel 1

### DNA Isolierung aus humanem Vollblut

Humanes Vollblut wird in ein Reaktionsgefäß pipettiert, welches 2,5 Volumen Lysepuffer (13 mM Tris-Cl; 10 mM KCl; 14,5 mM MgCl₂x6H₂O; 2 mM EDTA; 2,0% Triton X-100; pH 8) enthält. Nach 5-maligem Invertieren wird das Lysat abzentrifugiert, wodurch DNAenthaltende Zellkomponenten wie Zellkerne und Mitochondrien sedimentiert werden. Der Überstand wird verworfen. Anschließend wird das Sediment in Denaturierungspuffer (3 M Guanidinium HCl; 50 mM Tris-Cl pH 8,5) durch Vortexen oder Auf- und Abpipettieren homogenisiert. Proteine werden in einer 10 min Inkubation bei 65°C durch QIAGEN Protease entfernt. Aus dieser Lösung wird die DNA durch Zugabe von 1 Volumen Isopropanol präzipitiert und mittels Zentrifugation sedimentiert. Nach dem Abdekantieren des Überstandes wird das DNA-Pellet mit 70% Ethanol gewaschen, erneut abzentrifugiert, der Überstand vollständig entfernt, die DNA getrocknet und in einem Niedrigsalzpuffer oder Wasser resuspendiert.

Die folgende Tabelle 1 zeigt die Ergebnisse aus 6 parallel durchgeführten DNA-Aufreinigungen aus jeweils 3 ml humanem Vollblut, das mit Kalium-EDTA antikoaguliert wurde:

**Tabelle 1:**

| Präp.-Nr. | Ausbeute [µg] | A260/A280 |
|---|---|---|
| 1 | 62 | 1,84 |
| 2 | 76 | 1,92 |
| 3 | 67 | 1,94 |
| 4 | 68 | 1,93 |
| 5 | 66 | 1,92 |
| 6 | 58 | 1,95 |

Die mittlere Ausbeute der 6 Extraktionen beträgt 66 µg, dies entspricht 98% der nach Leukozyten-Zellzählung theoretisch zu erwartenden Ausbeute. Der Variationskoeffizient beträgt 8%.

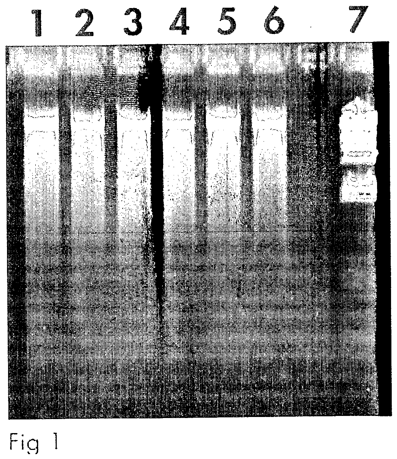

In Fig. 1 werden jeweils 2 µl der erhaltenen DNA Lösungen auf ein 0,8% Agarosegel aufgetragen.

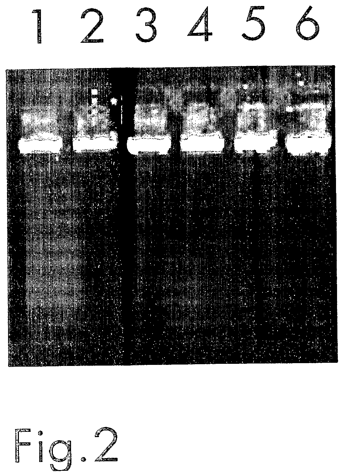

Fig. 2 zeigt die elektrophoretische Analyse einer Polymerase-Kettenreaktion (PCR) mit dem Ziel der Amplifikation eines Einzelkopie-Gens (human analog of giant larvae gene) unter Verwendung der aufgereinigten genomischen DNA als Matrize. Der Versuch zeigt, dass alle sechs Extraktionen reine DNA liefern, die problemlos amplifizierbar ist.

### Beispiel 2

### DNA Isolierung aus Buffy Coat

Die DNA Aufreinigung aus Buffy Coat erfolgt nach demselben Protokoll wie unter Beispiel 1 für Vollblut beschrieben. Im Unterschied zum Vollblutprotokoll wurden Tabelle 2 zeigt die Ergebnisse von 3x6 parallel durchgeführten Aufreinigungen mit ansteigenden Mengen (200; 300; 500 µl) Buffy Coat:

**Tabelle 2:**

| Vol. Buffy Coat | Präp.-Nr. | Ausbeute [µg] | A260/A280 | Mittelwert Ausbeute [µg] | Var. koeff. [%] |
|---|---|---|---|---|---|
| | 1 | 47 | 1,80 | | |
| | 2 | 52 | 1,82 | | |
| 200 µl | 3 | 50 | 1,80 | | |
| | 4 | 51 | 1,83 | | |
| | 5 | 53 | 1,81 | | |
| | 6 | 49 | 1,83 | 50 | 4 |
| | 7 | 74 | 1,83 | | |
| | 8 | 75 | 1,84 | | |
| 300 µl | 9 | 71 | 1,85 | | |
| | 10 | 75 | 1,84 | | |
| | 11 | 73 | 1,83 | | |
| | 12 | 70 | 1,85 | 73 | 3 |
| | 13 | 125 | 1,83 | | |
| | 14 | 111 | 1,85 | | |
| 500 µl | 15 | 118 | 1,84 | | |
| | 16 | 122 | 1,83 | | |
| | 17 | 118 | 1,84 | | |
| | 18 | 122 | 1,86 | 119 | 4 |

Der Versuch zeigt, dass die Ausbeute linear zum Ausgangsvolumen Buffy Coat ansteigt.

### Beispiel 3

### DNA Isolierung aus Zellkulturzellen

Die DNA Aufreinigung aus Zellkulturzellen (HeLa Zelllinie) erfolgt nach demselben Protokoll wie unter Beispiel 1 für Vollblut beschrieben.

Tabelle 3 zeigt die Ergebnisse aus 8 parallel durchgeführten DNA Aufreinigungen aus jeweils 1x10⁶ Zellen pro 200 µl PBS:

**Tabelle 3:**

| Präp.-Nr. | Ausbeute [µg] | A260/A280 |
|---|---|---|
| 1 | 21 | 1,91 |
| 2 | 20 | 1,91 |
| 3 | 20 | 1,92 |
| 4 | 21 | 1,93 |
| 5 | 19 | 1,94 |
| 6 | 19 | 1,94 |
| 7 | 18 | 1,93 |
| 8 | 19 | 1,95 |

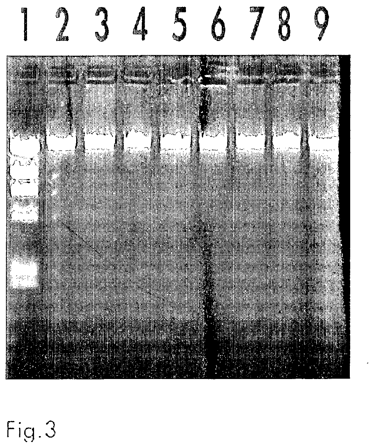

Die mittlere Ausbeute der 8 Extraktionen beträgt 20 µg. Der Variationskoeffizient beträgt 6%. In Fig. 3 wurden jeweils 15 µl der erhaltenen DNA Lösungen auf ein 0,8% Agarosegel aufgetragen.

### Beispiel 4

### DNA Isolierung aus humanem Vollblut mit verschiedenen Protokollvarianten

Die DNA Aufreinigung erfolgt wie in Beispiel 1 beschrieben. In einer Protokollvariante wird auf die Zugabe der QIAGEN Protease verzichtet. In einer zweiten Protokollvariante wird ebenfalls keine QIAGEN Protease zugegeben und darüber hinaus die Inkubation bei 65°C weggelassen. Alle weiteren Schritte der 3 Varianten sind identisch.
Tabelle 4 fasst die Ergebnisse dieses Versuches zusammen:

**Tabelle 4:**

| **+Protease/+65°C** | | | | | |
|---|---|---|---|---|---|
| Blutvolumen | Ausb. [%] | Ausb. CV [%] | 260/280 | 220/260 | hugl PCR [%] |
| ≤ 500 µl | 104 | 3 | 1,84 | 0,79 | 93 |
| ≤ 3 ml | 93 | 10 | 1,87 | 0,73 | 98 |
| ≤ 10 ml | 94 | 8 | 1,85 | 0,71 | 98 |
| Mittelwert | 97 | 7 | 1,85 | 0,75 | 97 |

| **-Protease/+65°C** | | | | | |
|---|---|---|---|---|---|
| Blutvolumen | Ausb. [%] | Ausb. CV [%] | 260/280 | 220/260 | hugl PCR [%] |
| ≤ 500 µl | 94 | 5 | 1,84 | 0,78 | 98 |
| ≤ 3 ml | 86 | 12 | 1,86 | 0,75 | 94 |
| ≤ 10 ml | 84 | 11 | 1,86 | 0,75 | 95 |
| Mittelwert | 88 | 9 | 1,85 | 0,76 | 96 |

| **-Protease/-65°C** | | | | | |
|---|---|---|---|---|---|
| Blutvolumen | Ausb. [%] | Ausb. CV [%] | 260/280 | 220/260 | hugl PCR [%] |
| ≤ 500 µl | 94 | 7 | 1,85 | 0,83 | 90 |
| ≤ 3 ml | 74 | 14 | 1,78 | 0,90 | 100 |
| ≤ 10 ml | 85 | 10 | 1,85 | 0,78 | 100 |
| Mittelwert | 84 | 10 | 1,83 | 0,84 | 97 |

Alle drei Protokollvarianten liefern DNA vergleichbarer Reinheit und Amplifizierbarkeit.

### Beispiel 5

### Verwendung von Lysereagenzien mit verschiedenen Salzen:

Aus humanem Vollblut wird gemäß Beispiel 1 genomische DNA aufgereinigt. Zur Lyse des Blutes werden Lysepuffer mit unterschiedlichen Salzen eingesetzt. Die folgende Tabelle fasst die verwendeten Lysepuffer-Alternativen mit den entsprechenden relativen Ausbeuten zusammen:

**Tabelle 5:**

| Puffer | Ausbeute [%] |
|---|---|
| 13 mM Tris-Cl; 10 mM KCl; 14,5 mM MgCl₂; 2 mM EDTA; 2% Triton X-100; pH 8 | 100 |
| 20 mM Tris-Cl; 10 mM KCl; 5/10 mM MgCl₂; 2 mM EDTA; 2% Triton X-100 | 105/96 |
| 20 mM Tris-Cl; 10 mM KCl; 14,5 mM MgCl₂; 10/20/50 mM (NH₄)₂SO₄; 2 mM EDTA; 2% Triton X-100 | 108/103/103 |
| 20 mM Tris-Cl; 10 mM KCl; 15 mM MgCl₂; 150/200/250 mM (NH₄)₂SO₄; 2 mM EDTA; 2% Triton X-100 | 102/93/97 |
| 20 mM Tris-Cl; 10 mM KCl; 1/2,5 mM ZnCl₂; 2 mM EDTA; 2% Triton X-100 | 108/88 |
| 20 mM Tris-Cl; 10 mM KCl; 1/2,5/3/4/5 mM ZnCl₂; 5 mM MgCl₂; 2 mM EDTA; 2% Triton X-100 | 91/94/96/100/99 |
| 20 mM Tris-Cl; 10 mM KCl; 15 mM CaCl₂; 2 mM EDTA; 2% Triton X-100 | 111 |
| 20 mM Tris-Cl; 10 mM KCl; 10 mM CaCl₂; 2 mM MgCl₂ ; 2 mM EDTA; 2% Triton X-100 | 104 |

### Beispiel 6

### Verwendung von Lysereagenzien mit verschiedenen Puffersubstanzen/pH-Werten:

Aus humanem Vollblut wird gemäß Beispiel 1 genomische DNA aufgereinigt. Zur Lyse des Blutes werden Lysepuffer mit unterschiedlichen Puffersubstanzen/pH-Werten eingesetzt. Die folgende Tabelle faßt die verwendeten Lysepufferalternativen mit den entsprechenden relativen Ausbeuten zusammen:

**Tabelle 6:**

| Puffer | Ausbeute [%] |
|---|---|
| 13 mM Tris-Cl pH 8,0; 10 mM KCl; 14,5 mM MgCl₂; 2 mM EDTA; 2% Triton X-100 | 100 |
| 50/100 mM Na-Acetat pH 4,0; 10 mM KCI; 15 mM MgCl₂; 2 mM EDTA; 2% Triton X-100 | 107/110 |
| 50/100 mM Glycin-HCl pH 3,0; 10 mM KCl; 15 mM MgCl₂; 2 mM EDTA; 2% Triton X-100 | 99/106 |
| 50/100/160 mM Lysin-HCl pH 3,0/3,3/3,4; 10 mM KCl; 15 mM MgCl₂; 4 mM EGTA; 2% Triton X-100 | 103/107/124 |
| 50/100/160 mM Lysin-HCl pH 3,3/3,5/3,7; 10 mM KCl; 15 mM MgCl₂; 2 mM EGTA; 2% Triton X-100 | 109/101/100 |
| 50/100/160 mM Lysin-HCl pH 4,3/4,7/4,6; 10 mM KCl; 15 mM MgCl₂; 2% Triton X-100 | 109/116/113 |
| 50 mM Ornithin-HCl pH 3,0; 10 mM KCI; 14,5 mM MgCl₂; 2 mM EGTA; 2% Triton X-100 | 111 |

### Beispiel 7

### Verwendung von Lysereagenzien mit verschiedenen Komplexbildnern:

Aus humanem Vollblut wird gemäß Beispiel 1 genomische DNA aufgereinigt. Zur Lyse des Blutes werden Lysepuffer mit unterschiedlichen Komplexbildnern eingesetzt. Die folgende Tabelle faßt die verwendeten Lysepufferalternativen mit den entsprechenden relativen Ausbeuten zusammen (EGTA = Ethylendioxy-bis-(ethylennitrilo)-tetra-essigsäure; DTPA = Diethylentriamin-penta-essigsäure):

**Tabelle 7:**

| Puffer | Ausbeute [%] |
|---|---|
| 13 mM Tris-Cl pH 8,0; 10 mM KCl; 14,5 mM MgCl₂; 2 mM EDTA; 2% Triton X-100 | 100 |
| 13 mM Tris-Cl pH 8,0; 10 mM KCl; 14,5 mM MgCl₂; 3/4/4,5/5 mM EDTA; 2% Triton X-100 | 97/113/101/103 |
| 13 mM Tris-Cl pH 8,0; 10 mM KCl; 14,5 mM MgCl₂; 1 mM EGTA; 2% Triton X-100 | 124 |
| 13 mM Tris-Cl pH 8,0; 10 mM KCl; 14,5 mM MgCl₂; 2 mM EDTA; 1 mM EGTA; 2% Triton X-100 | 106 |
| 13 mM Tris-Cl pH 8,0; 10 mM KCl; 14,5 mM MgCl₂; 1/2 mM DTPA; 2% Triton X-100 | 101/99 |
| 13 mM Tris-Cl pH 8,0; 10 mM KCl; 14,5 mM MgCl₂; 1/2 mM DTPA; 2 mM EDTA; 2% Triton X-100 | 95/102 |
| 13 mM Tris-Cl pH 8,0; 10 mM KCl; 14,5 mM MgCl₂; 1/2 mM Na₅P₃O₁₀; 2% Triton X-100 | 79/197 |
| 13 mM Tris-Cl pH 8,0; 10 mM KCl; 14,5 mM MgCl₂; 1/2 mM Na₅P₃O₁₀; 2 mM EDTA; 2% Triton X-100 | 95/92 |
| 20 mM Tris-Cl pH 8,0; 10 mM KCl; 15 mM MgCl₂; 1/2 mM EGTA; 1% Triton X-100 | 110/102 |
| 20 mM Tris-Cl pH 8,0; 10 mM KCl; 15 mM MgCl₂; 2/4 mM EGTA; 2% Triton X-100 | 111/140 |

### Beispiel 8

### Verwendung von Lysereagenzien mit verschiedenen Detergenzkonzentrationen:

Aus humanem Vollblut wird gemäß Beispiel 1 genomische DNA aufgereinigt. Zur Lyse des Blutes werden Lysepuffer mit unterschiedlichen Detergenzkonzentrationen eingesetzt. Die folgende Tabelle faßt die verwendeten Lysepufferalternativen mit den entsprechenden relativen Ausbeuten zusammen:

**Tabelle 8:**

| Puffer | Ausbeute [%] |
|---|---|
| 13 mM Tris-Cl pH 8,0; 10 mM KCl; 14,5 mM MgCl₂; 2 mM EDTA; 1/2/2,5/3/4% Triton X-100 | 103/100/103/102/98 |

### Beispiel 9

### Verwendung von Salzlösungen zum Resuspendieren des DNA-haltigen Materials mit verschiedenen Puffersubstanzen/pH-Werten:

Aus humanem Vollblut wird gemäß Beispiel 1 genomische DNA aufgereinigt. Als Lysereagenz wird 13 mM Tris-Cl pH 8,0; 10 mM KCl; 14,5 mM MgCl₂; 1 mM EGTA; 2% Triton X-100 eingesetzt. Zum Resuspendieren des DNA-haltigen Materials werden Salzlösungen mit unterschiedlichen Puffersubstanzen/pH-Werten eingesetzt. Die folgende Tabelle faßt die verwendeten Alternativen mit den entsprechenden relativen Ausbeuten zusammen:

**Tabelle 9**

| Puffer | Ausbeute [%] |
|---|---|
| 3 M Guanidinium hydrochlorid; 50 mM Tris-Cl pH 8,5 | 100 |
| 3 M Guanidinium hydrochlorid; 5 mM CaCl₂; 50 mM Tris-Cl pH 8,5 | 118 |
| 3 M Guanidinium hydrochlorid; 5 mM CaCl₂; 50 mM Tris pH 9,5 | 129 |
| 3 M Guanidinium hydrochlorid; 50 mM Glycin-NaOH pH 10,0 | 124 |
| 3 M Guanidinium hydrochlorid; 5 mM CaCl₂; 50 mM Glycin-NaOH pH 10,0 | 124 |

### Beispiel 10

### Verwendung von Salzlösungen zum Resuspendieren des DNA-haltigen Materials mit verschiedenen chaotropen Salzen:

Aus humanem Vollblut wird gemäß Beispiel 1 genomische DNA aufgereinigt. Als Lysereagenz wird 13 mM Tris-Cl pH 8,0; 10 mM KCl; 14,5 mM MgCl₂; 1 mM EGTA; 2% Triton X-100 eingesetzt. Zum Resuspendieren des DNA-haltigen Materials werden Salzlösungen mit verschiedenen Chaotropen eingesetzt. Die folgende Tabelle faßt die verwendeten Alternativen mit den entsprechenden relativen Ausbeuten zusammen:

**Tabelle 10:**

| Puffer | Ausbeute [%] |
|---|---|
| 3 M Guanidinium hydrochlorid; 50 mM Tris-Cl pH 8,5 | 100 |
| 3 M Guanidinium hydrochlorid; 0,1 M Harnstoff; 50 mM Tris-Cl pH 8,5 | 100 |
| 2,5 M Guanidinium hydrochlorid; 0,5 M Harnstoff; 50 mM Tris-Cl pH 8,5 | 98 |
| 2 M Guanidinium hydrochlorid; 1 M Harnstoff; 50 mM Tris-Cl pH 8,5 | 94 |
| 1,5 M Guanidinium hydrochlorid; 1,5 M Harnstoff; 50 mM Tris-Cl pH 8,5 | 101 |
| 1,5 M Harnstoff; 50 mM Tris-Cl pH 8,5 | 86 |

### Beispiel 11

### Unterschiedliche Temperaturen während der Protease-Inkubation

Aus humanem Vollblut wird gemäß Beispiel 1 genomische DNA aufgereinigt. Als Lysereagenz wird 13 mM Tris-Cl pH 8,0; 10 mM KCl; 14,5 mM MgCl₂; 1 mM EGTA; 2% Triton X-100 eingesetzt. Die Inkubation mit Protease erfolgte für 5 bzw. 10 min bei verschiedenen Temperaturen. Die folgende Tabelle fasst die Ergebnisse zusammen:

**Tabelle 11:**

| Inkubation | Ausbeute [%] |
|---|---|
| 5 min 65°C | 100 |
| 10 min 65°C | 99 |
| 5 min 75°C | 95 |
| 10 min 75°C | 85 |
| 5 min 85°C | 84 |
| 10 min 85°C | 70 |

### Beispiel 12

### Lipase-Inkubation

Aus humanem Vollblut wird gemäß Beispiel 1 genomische DNA aufgereinigt. Als Lysereagenz wird a) 13 mM Tris-CI pH 8,0; 10 mM KCI; 14,5 mM MgCl₂; 2 mM EDTA; 1 mM EGTA; 2% Triton X-100 bzw. b) 13 mM Tris-Cl pH 8,0; 10 mM KCl; 14,5 mM MgCl₂; 2 mM EDTA; 2% Triton X-100 eingesetzt. Varianten mit und ohne Lipase-Inkubation (5 min 60°C) vor der Protease-Inkubation werden verglichen:

**Tabelle 12:**

| Inkubation | Ausbeute [%] |
|---|---|
| a) -Lipase | 100 |
| a) +Lipase | 93 |
| b) -Lipase | 100 |
| b) +Lipase | 91 |

## Patentansprüche

1. Verfahren zur Isolierung von DNA, bei dem man eine biologische Probe mit einem Lysereagenz vermischt, das Salze einwertiger Kationen, Salze zweiwertiger Kationen, einen oder mehrere Komplexbildner ausgewählt aus Ethylendiamintetraacetat, Ethylendioxy-bis-(ethylennitrilo)tetraessigsäure, DTPA und Na₅P₃O₁₀ und Octylphenol polyethoxylat enthält, danach die DNA-enthaltenden Zellkomponenten von den übrigen Zellkomponenten trennt, das DNA-haltige Zellmaterial in einer Salzlösung resuspendiert, wobei die Salzlösung mindestens ein chaotropes Salz enthält, gegebenenfalls kontaminierende Bestandteile durch eine chemische, thermische und/oder enzymatische Behandlung abtrennt, die DNA präzipitiert, wäscht, trocknet und ggf. in einer Niedrigsalzlösung resuspendiert, wobei die Isolierung in einem Reaktionsgefäß durchgeführt wird.

2. Verfahren nach Anspruch 1, **dadurch gekennzeichnet, dass** das Lysereagenz 0,5 bis 7,5 Vol.-%, vorzugsweise 1,0 bis 5,0 Vol.-% Detergenz enthält.

3. Verfahren nach einem der Ansprüche 1 oder 2, **dadurch gekennzeichnet, dass** das Lysereagenz 0,5 bis 200 mM Magnesiumchlorid und/oder 0,5 bis 200 mM Kaliumchlorid und/oder 0,5 bis 100 mM Ethylendiamintetraacetat und/oder 0,5 bis 100 mM Ethylendioxy-bis-(ethylennitrilo)tetraessigsäure und besonders bevorzugt 1 bis 30 mM Magnesiumchlorid und/oder 1 bis 20 mM Kaliumchlorid und/oder 1 bis 10 mM Ethylendiamintetraacetat und/oder 1 bis 10 mM Ethylendioxy-bis(ethylennitrilo)tetraessigsäure enthalten kann.

4. Verfahren nach Anspruch 1, **dadurch gekennzeichnet, dass** das Lysereagenz des Weiteren eine oder mehrere Puffersubstanzen enthalten kann.

5. Verfahren nach Anspruch 4, **dadurch gekennzeichnet, dass** als Puffersystem Tris(hydroxymethyl)aminomethan-Hydrochlorid, Natriumacetat, Glycin-Hydrochlorid, Lysin-Hydrochlorid oder Ornithin-Hydrochlorid, bevorzugt in einer Konzentration von 0,5 bis 250 mM, eingesetzt werden kann.

6. Verfahren nach einem der Ansprüche 4 oder 5, **dadurch gekennzeichnet, dass** der pH-Wert des Lysepuffers in einem Bereich von 2 bis 10 und bevorzugt in einem Bereich von 3 bis 9 liegt.

7. Verfahren nach einem der Ansprüche 1, 4, 5 oder 6, **dadurch gekennzeichnet, dass** der Lysepuffer 13 mM Tris(hydroxymethyl)aminomethan Hydrochlorid, pH 8,0, 10 bis 14,5 mM Magnesium(II)chlorid Hexahydrat, 10 mM Kaliumchlorid, 2 bis 5 mM Ethylendiamintetraacetat und/oder 1 bis 2 mM Ethylendioxy-bis(ethylennitrilo)tetraessigsäure sowie 2 Vol.-% Octylphenol polyethoxylat in wässeriger Lösung enthält.

8. Verfahren nach Anspruch 1, **dadurch gekennzeichnet, dass** die Salzlösung zum Resuspendieren des DNA-haltigen Materials Harnstoff, Guanidinium-*iso*-Thiocyanat oder Guanidinium-Hydrochlorid oder eine Mischung dieser Salze enthält.

9. Verfahren nach Anspruch 8, **dadurch gekennzeichnet, dass** die Salzlösung zum Resuspendieren des DNA-haltigen Materials eine wässerige, 0,5 bis 7,0 M Guanidinium-Hydrochlorid-Lösung ist, die gegebenenfalls als weitere Hilfsstoffe Puffersubstanzen, Detergenzien oder Komplexbildner enthalten kann.

10. Verfahren nach Anspruch 9, **dadurch gekennzeichnet, dass** die Salzlösung zum Resuspendieren des DNA-haltigen Materials eine wässerige, 2,0 bis 4,0 M Guanidinium-Hydrochlorid-Lösung ist, die gegebenenfalls weitere Hilfsstoffe wie Puffersubstanzen, Detergenzien oder Komplexbildner enthalten kann.

11. Verfahren nach Anspruch 9 oder 10, **dadurch gekennzeichnet, dass** die Salzlösung ein auf Tris(hydroxymethyl)aminomethan- oder Glycin-Natriumhydroxid basierendes Puffersystem, vorzugsweise in einer Konzentration von 0,5 bis 250 mM enthält.

12. Verfahren nach einem der Ansprüche 1 oder 10 bis 11, **dadurch gekennzeichnet, dass** der pH-Wert der gepufferten Salzlösung in einem Intervall von 3 bis 10, vorzugsweise in einem Intervall von 7,5 bis 9,5, liegt.

13. Verfahren nach einem der Ansprüche 1 oder 10 bis 12, **dadurch gekennzeichnet, dass** sich die gepufferte Salzlösung zum Resuspendieren des DNA-haltigen Materials im wesentlichen aus einer wässerigen, 3 M Guanidinium-Hydrochlorid-Lösung und 50 mM Tris(hydroxymethyl)aminomethan Hydrochlorid, pH 8,5 zusammensetzt.

14. Verfahren nach Anspruch 1, **dadurch gekennzeichnet, dass** man zur Abtrennung von Kontaminationen die Probe erwärmt, bevorzugt auf eine Temperatur in einem Intervall von 15 bis 95 °C und besonders bevorzugt auf eine Temperatur in einem Intervall von 30 bis 85 °C.

15. Verfahren nach Anspruch 1, **dadurch gekennzeichnet, dass** man zur Degradation von Verunreinigungen Enzyme, bevorzugt Enzyme aus der Gruppe der Proteasen, Lipasen, Amylasen oder Enzymmischungen einsetzt.

16. Verfahren nach Anspruch 1, **dadurch gekennzeichnet, dass** man die Nukleinsäure durch Zugabe eines verzweigten oder unverzweigten C₁-C₄- Alkanols, bevorzugt Propan-2-ol (*iso*-Propanol), oder von Gemischen dieser Alkohole präzipitiert.

17. Verfahren nach Anspruch 1, **dadurch gekennzeichnet, dass** man als Waschflüssigkeit eine Mischung aus einem verzweigten oder unverzweigten C₁-C₄-Alkanol und Wasser, bevorzugt eine Mischung aus einer Lösung von Ethanol in Wasser und besonders bevorzugt eine 70 Vol.-prozentige Lösung von Ethanol in Wasser einsetzt.

18. Verfahren nach Anspruch 1, **dadurch gekennzeichnet, dass** man als Niedrigsalzlösung ein Lösungsmittel oder eine Salzlösung einsetzt, in der sich die DNA löst.

19. Verfahren nach Anspruch 18, **dadurch gekennzeichnet, dass** als Lösungsmittel Wasser eingesetzt wird.

20. Verfahren nach Anspruch 18 oder 19, **dadurch gekennzeichnet, dass** die Niedrigsalzlösung oder das Lösungsmittel des weiteren Puffersubstanzen, bevorzugt einen auf Tris(hydroxymethyl)aminomethan basierenden Puffer und besonders bevorzugt bis 150 mM Tris(hydroxymethyl)aminomethan Hydrochlorid und/oder gegebenenfalls Komplexbildner enthält.

21. Verfahren nach Anspruch 20, **dadurch gekennzeichnet, dass** der pH-Wert des Niedrigsalzpuffers in einem Intervall von 6 bis 10 und vorzugsweise in einem Intervall von 7.5 bis 9.5 liegt.

22. Verfahren nach Anspruch 21, **dadurch gekennzeichnet, dass** als Niedrigsalzlösung eine wässrige Lösung aus 10mM Tris(hydroxymethyl)aminomethan-Hydrochlorid mit einem pH-Wert von 8,5 verwendet wird.

23. Verfahren nach einem der Ansprüche 1 bis 22, **dadurch gekennzeichnet, dass** man als biologische Probe humanes Vollblut einsetzt.

24. Verfahren nach einem der Ansprüche 1 bis 22, **dadurch gekennzeichnet, dass** man als biologische Probe Buffy Coat einsetzt.

25. Verfahren nach einem der Ansprüche 1 bis 22, **dadurch gekennzeichnet, dass** man als biologische Probe eine Zellkultur einsetzt.

26. Kit enthaltend Reagenzien, auch in konzentrierter Form, zur fertigen Abmischung beim Anwender enthaltend die folgenden wässrigen Lösungen:
a) Octylphenol polyethoxylat sowie Salze einwertiger Kationen, Salze zweiwertiger Kationen und einen oder mehrere Komplexbildner ausgewählt aus Ethylendiamintetraacetat, Ethylendioxy-bis-(ethylennitrilo)tetraessigsäure, DTPA und Na₅P₃O₁₀, vorzugsweise 0,5 bis 200 mM Magnesium(II)chlorid und/oder 0,5 bis 200 mM Kaliumchlorid und/oder 0,5 bis 100 mM Ethylendiamintetraacetat und/oder 0,5 bis 100 mM Ethylendioxy-bis(ethylennitrilo)tetraessigsäure und besonders bevorzugt 1 bis 30 mM Magnesiumchlorid und/oder 1 bis 20 mM Kaliumchlorid und/oder 1 bis 10 mM Ethylendiamintetraacetat und/oder 1 bis 10 mM Ethylendioxy-bis-(ethylennitrilo)-tetraessigsäure und gegebenenfalls ein Puffersystem auf der Basis Tris(hydroxymethyl)aminomethan-Hydrochlorid, Natrium-Acetat, Glycin-Hydrochlorid, Lysin-Hydrochlorid oder Ornithin-Hydrochlorid, bevorzugt in einer Konzentration von 0,5 bis 250 mM;
b) eine Salzlösung zum Resuspendieren des DNA-haltigen Materials, die mindestens ein chaotropes Salz enthält, bevorzugt Harnstoff, Guanidinium-*iso*-Thiocyanat oder Guanidinium-Hydrochlorid oder eine Mischung beider dieser Salze;
c) gegebenenfalls Enzyme zur Degradation von Verunreingungen, bevorzugt Enzyme aus der Gruppe der Proteasen, Lipasen, Amylasen oder Enzymmischungen.
d) einen verzweigten oder unverzweigten C₁-C₄- Alkohol, bevorzugt Propan-2-ol (*iso-*Propanol), oder Gemische dieser Alkohole zur Präzipitation der Nukleinsäuren;
e) gegebenenfalls eine Waschflüssigkeit bestehend aus einer Mischung aus einem verzweigten oder unverzweigten C₁-C₄-Alkanol und Wasser, bevorzugt eine Mischung aus einer Lösung von Ethanol in Wasser und besonders bevorzugt eine 70 Vol.-prozentige Lösung von Ethanol in Wasser;
f) eine Niedrigsalzlösung oder ein Lösungsmittel, in dem sich die Nukleinsäuren lösen sowie gegebenenfalls Puffer, die bevorzugt einen auf Tris(hydroxymethyl)aminomethan und besonders bevorzugt bis 150 mM Tris(hydroxymethyl)aminomethan Hydrochlorid basieren und/oder gegebenenfalls Komplexbildner.

## Claims

1. Method for isolating DNA in which a biological sample is mixed with a lysis reagent which comprises salts of monovalent cations, salts of divalent cations, one or more complexing agents selected from ethylenediaminetetraacetate, ethylenedioxybis(ethylene-nitrilo)tetraacetic acid, DTPA and Na₅P₃O₁₀ and octylphenol polyethoxylate then the DNA-containing cell components are separated from the other cell components, the DNA-containing cell material is resuspended in a salt solution, wherein the salt solution comprises at least one chaotropic salt, where appropriate contaminating constituents are removed by a chemical, thermal and/or enzymatic treatment, the DNA is precipitated, washed, dried and, where appropriate, resuspended in a low-salt solution, wherein the isolation is carried out in one reaction vessel.

2. Method according to Claim 1, **characterized in that** the lysis reagent comprises from 0.5 to 7.5% by volume, preferably 1.0 to 5.0% by volume, of detergent.

3. Method according to either of Claims 1 or 2, **characterized in that** the lysis reagent may comprise 0.5 to 200 mM magnesium chloride and/or 0.5 to 200 mM potassium chloride and/or 0.5 to 100 mM ethylenediaminetetraacetate and/or 0.5 to 100 mM ethylenedioxybis(ethylenenitrilo)tetraacetic acid and particularly preferably 1 to 30 mM magnesium chloride and/or 1 to 20 mM potassium chloride and/or 1 to 10 mM ethylenediaminetetraacetate and/or 1 to 10 mM ethylenedioxybis(ethylenenitrilo)tetraacetic acid.

4. Method according to Claim 1, **characterized in that** the lysis reagent may further comprise one or more buffer substances.

5. Method according to Claim 4, **characterized in that** tris(hydroxymethyl)aminomethane hydrochloride, sodium acetate, glycine hydrochloride, lysine hydrochloride or ornithine hydrochloride, preferably in a concentration of from 0.5 to 250 mM, can be employed as buffer system.

6. Method according to either of Claims 4 or 5, **characterized in that** the pH value of the lysis buffer is in a range of from 2 to 10 and preferably in a range of from 3 to 9.

7. Method according to any of Claims 1, 4, 5 or 6, **characterized in that** the lysis buffer comprises 13 mM tris(hydroxymethyl)aminomethane hydrochloride, pH 8.0, 10 to 14.5 mM magnesium(II) chloride hexahydrate, 10 mM potassium chloride, 2 to 5 mM ethylenediaminetetraacetate and/or 1 to 2 mM ethylenedioxybis(ethylenenitrilo)tetraacetic acid and 2% by volume of octylphenol polyethoxylate in aqueous solution.

8. Method according to Claim 1, **characterized in that** the salt solution for resuspending the DNA-containing material comprises urea, guanidinium *iso*-thiocyanate or guanidinium hydrochloride or a mixture of these salts.

9. Method according to Claim 8, **characterized in that** the salt solution for resuspending the DNA-containing material is an aqueous 0.5 to 7.0 M guanidinium hydrochloride solution which may optionally comprise as further auxiliaries buffer substances, detergents or complexing agents.

10. Method according to Claim 9, **characterized in that** the salt solution for resuspending the DNA-containing material is an aqueous 2.0 to 4.0 M guanidinium hydrochloride solution which may optionally comprise further auxiliaries like buffer substances, detergents or complexing agents.

11. Method according to Claim 9 or 10, **characterized in that** the salt solution comprises a buffer system based on tris(hydroxymethyl)aminomethane or glycine-sodium hydroxide, preferably in a concentration of from 0.5 to 250 mM.

12. Method according to any of Claims 1 or 10 to 11, **characterized in that** the pH of the buffered salt solution is in a range of from 3 to 10, preferably in a range of from 7.5 to 9.5.

13. Method according to any of Claims 1 or 10 to 12, **characterized in that** the buffered salt solution for resuspending the DNA-containing material is essentially composed of an aqueous 3 M guanidinium hydrochloride solution and 50 mM tris(hydroxymethyl)aminomethane hydrochloride, pH 8.5.

14. Method according to Claim 1, **characterized in that** contaminants are removed by heating the sample, preferably to a temperature in a range of from 15 to 95°C and particularly preferably to a temperature in a range of from 30 to 85°C.

15. Method according to Claim 1, **characterized in that** enzymes, preferably enzymes from the group of proteases, lipases, amylases or enzyme mixtures are employed to degrade impurities.

16. Method according to Claim 1, **characterized in that** the nucleic acid is precipitated by adding a branched or unbranched C₁-C₄-alkanol, preferably propan-2-ol (*iso*-propanol), or mixtures of these alcohols.

17. Method according to Claim 1, **characterized in that** the washing liquid employed is a mixture of a branched or unbranched C₁-C₄-alkanol and water, preferably a mixture of a solution of ethanol in water and particularly preferably a 70% by volume solution of ethanol in water.

18. Method according to Claim 1, **characterized in that** a solvent or a salt solution in which DNA dissolves is employed as low-salt solution.

19. Method according to Claim 18, **characterized in that** water is employed as solvent.

20. Method according to Claim 18 or 19, **characterized in that** the low-salt solution or the solvent further comprises buffer substances, preferably a buffer based on tris(hydroxymethyl)aminomethane and particularly preferably up to 150 mM tris(hydroxymethyl)aminomethane hydrochloride and/or optionally complexing agents.

21. Method according to Claim 20, **characterized in that** the pH value of the low-salt buffer is in a range from 6 to 10 and preferably in a range from 7.5 to 9.5.

22. Method according to Claim 21, **characterized in that** an aqueous solution of 10 mM tris(hydroxymethyl)aminomethane hydrochloride with a pH value of 8.5 is used as low-salt solution.

23. Method according to any of Claims 1 to 22, **characterized in that** human whole blood is employed as biological sample.

24. Method according to any of Claims 1 to 22, **characterized in that** buffy coat is employed as biological sample.

25. Method according to any of Claims 1 to 22, **characterized in that** a cell culture is employed as biological sample.

26. Kit comprising reagents, also in concentrated form, ready for mixing by the user, comprising the following aqueous solutions:
a) octylphenol polyethoxylate and salts of monovalent cations, salts of divalent cations and one or more complexing agents selected from ethylenediaminetetraacetate, ethylenedioxybis-(ethylenenitrilo)tetraacetic acid, DTPA and Na₅P₃O₁₀, preferably 0.5 to 200 mM magnesium(II) chloride and/or 0.5 to 200 mM potassium chloride and/or 0.5 to 100 mM ethylenediaminetetraacetate and/or 0.5 to 100 mM ethylenedioxybis(ethylenenitrilo)tetraacetic acid and particularly preferably 1 to 30 mM magnesium chloride and/or 1 to 20 mM potassium chloride and/or 1 to 10 mM ethylenediaminetetraacetate and/or 1 to 10 mM ethylenedioxybis(ethylenenitrilo)tetraacetic acid, and where appropriate a buffer system based on tris(hydroxymethyl)aminomethane hydrochloride, sodium acetate, glycine hydrochloride, lysine hydrochloride or ornithine hydrochloride, preferably in a concentration of from 0.5 to 250 mM;
b) a salt solution for resuspending the DNA-containing material which contains at least one chaotropic salt, preferably urea, guanidinium *iso-*thiocyanate or guanidinium hydrochloride or a mixture of both of these salts;
c) where appropriate enzymes to degrade impurities, preferably enzymes from the group of proteases, lipases, amylases or enzyme mixtures.
d) a branched or unbranched C₁-C₄-alcohol, preferably propan-2-ol (*iso*-propanol), or mixtures of these alcohols for precipitating the nucleic acids;
e) where appropriate a washing liquid consisting of a mixture of a branched or unbranched C₁-C₄-alkanol and water, preferably a mixture of a solution of ethanol in water and particularly preferably a 70% by volume solution of ethanol in water;
f) a low-salt solution or a solvent in which the nucleic acids dissolve, and where appropriate buffers which are preferably based on tris(hydroxymethyl)aminomethane and particularly preferably up to 150 mM tris(hydroxymethyl)aminomethane hydrochloride and/or where appropriate complexing agents.

## Revendications

1. Procédé d'isolement d'ADN, selon lequel un échantillon biologique est mélangé avec un réactif de lyse, qui contient des sels de cations monovalents, des sels de cations bivalents, un ou plusieurs complexants choisis parmi le tétraacétate d'éthylène diamine, l'acide éthylènedioxy-bis-(éthylène-nitrilo)tétraacétique, le DTPA et le Na₅P₃O₁₀ et le polyéthoxylate d'octylphénol, puis les composants cellulaires contenant de l'ADN sont séparés des autres composants cellulaires, le matériau cellulaire contenant de l'ADN est resuspendu dans une solution saline, la solution saline contenant au moins un sel chaotropique, des constituants contaminants sont éventuellement séparés par un traitement chimique, thermique et/ou enzymatique, l'ADN est précipité, lavé, séché et éventuellement resuspendu dans une solution saline faible, l'isolement étant réalisé dans un contenant de réaction.

2. Procédé selon l'une quelconque de la revendication 1, **caractérisé en ce que** le réactif de lyse contient 0,5 à 7,5 % en volume, de préférence 1,0 à 5,0 % en volume, de détergent.

3. Procédé selon l'une quelconque des revendications 1 ou 2, **caractérisé en ce que** le réactif de lyse peut contenir 0,5 à 200 mM de chlorure de magnésium et/ou 0,5 à 200 mM de chlorure de potassium et/ou 0,5 à 100 mM de tétraacétate d'éthylène-diamine et/ou 0,5 à 100 mM d'acide éthylènedioxy-bis-(éthylène-nitrilo)tétraacétique, et de manière particulièrement préférée 1 à 30 mM de chlorure de magnésium et/ou 1 à 20 mM de chlorure de potassium et/ou 1 à 10 mM de tétraacétate d'éthylène-diamine et/ou 1 à 10 mM d'acide éthylènedioxy-bis-(éthylène-nitrilo)tétraacétique.

4. Procédé selon la revendication 1, **caractérisé en ce que** le réactif de lyse peut en outre contenir une ou plusieurs substances tampon.

5. Procédé selon la revendication 4, **caractérisé en ce que** du chlorhydrate de tris(hydroxyméthyl)aminométhane, de l'acétate de sodium, du chlorhydrate de glycine, du chlorhydrate de lysine ou du chlorhydrate d'ornithine peut être utilisé en tant que système tampon, de préférence en une concentration de 0,5 à 250 mM.

6. Procédé selon l'une quelconque des revendications 4 ou 5, **caractérisé en ce que** le pH du tampon de lyse se situe dans une plage allant de 2 à 10 et de préférence dans une plage allant de 3 à 9.

7. Procédé selon l'une quelconque des revendications 1, 4, 5 ou 6, **caractérisé en ce que** le tampon de lyse contient 13 mM de chlorhydrate de tris(hydroxyméthyl)aminométhane, pH 8,0, 10 à 14,5 mM d'hexahydrate de chlorure de magnésium (II), 10 mM de chlorure de potassium, 2 à 5 mM de tétraacétate d'éthylène-diamine et/ou 1 à 2 mM d'acide éthylènedioxy-bis-(éthylène-nitrilo)tétraacétique, ainsi que 2 % en volume de polyéthoxylate d'octylphénol en solution aqueuse.

8. Procédé selon la revendication 1, **caractérisé en ce que** la solution saline pour la resuspension du matériau contenant de l'ADN contient de l'urée, de l'*iso-*thiocyanate de guanidinium ou du chlorhydrate de guanidinium ou un mélange de ces sels.

9. Procédé selon la revendication 8, **caractérisé en ce que** la solution saline pour la resuspension du matériau contenant de l'ADN est une solution aqueuse de chlorhydrate de guanidinium de 0,5 à 7,0 M, qui peut éventuellement contenir en tant qu'adjuvants supplémentaires des substances tampon, des détergents ou des complexants.

10. Procédé selon la revendication 9, **caractérisé en ce que** la solution saline pour la resuspension du matériau contenant de l'ADN est une solution aqueuse de chlorhydrate de guanidinium de 2,0 à 4,0 M, qui peut éventuellement contenir en tant qu'adjuvants supplémentaires des substances tampon, des détergents ou des complexants.

11. Procédé selon la revendication 9 ou 10, **caractérisé en ce que** la solution saline contient un système tampon à base d'hydroxyde de tris(hydroxyméthyl)aminométhane ou de glycine-sodium, de préférence en une concentration de 0,5 à 250 mM.

12. Procédé selon l'une quelconque des revendications 1 ou 10 à 11, **caractérisé en ce que** le pH de la solution saline tamponnée se situe dans un intervalle allant de 3 à 10, de préférence dans un intervalle allant de 7,5 à 9,5.

13. Procédé selon l'une quelconque des revendications 1 ou 10 à 12, **caractérisé en ce que** la solution saline tamponnée pour la resuspension du matériau contenant de l'ADN se compose essentiellement d'une solution aqueuse de chlorhydrate de guanidinium à 3 M et de chlorhydrate de tris(hydroxyméthyl)aminométhane à 50 mM, pH 8,5.

14. Procédé selon la revendication 1, **caractérisé en ce que** l'échantillon est chauffé pour la séparation de contaminations, de préférence à une température dans un intervalle allant de 15 à 95 °C et de manière particulièrement préférée à une température dans un intervalle allant de 30 à 85 °C.

15. Procédé selon la revendication 1, **caractérisé en ce que** des enzymes sont utilisées pour la dégradation d'impuretés, de préférence des enzymes du groupe constitué par les protéases, les lipases, les amylases ou des mélanges d'enzymes.

16. Procédé selon la revendication 1, **caractérisé en ce que** l'acide nucléique est précipité par ajout d'un alcanol en C₁-C₄ ramifié ou non ramifié, de préférence le propan-2-ol (*iso*-propanol) ou des mélanges de ces alcools.

17. Procédé selon la revendication 1, **caractérisé en ce qu'**un mélange d'un alcanol en C₁-C₄ ramifié ou non ramifié et d'eau, de préférence un mélange d'une solution d'éthanol dans de l'eau et de manière particulièrement préférée une solution à 70 pour cent en volume d'éthanol dans de l'eau, est utilisé en tant que liquide de lavage.

18. Procédé selon la revendication 1, **caractérisé en ce qu'**un solvant ou une solution saline dans lequel ou dans laquelle l'ADN se dissous est utilisé en tant que solution saline faible.

19. Procédé selon la revendication 18, **caractérisé en ce que** de l'eau est utilisée en tant que solvant.

20. Procédé selon la revendication 18 ou 19, **caractérisé en ce que** la solution saline faible ou le solvant contient en outre des substances tampon, de préférence un tampon à base de tris(hydroxyméthyl)aminométhane et de manière particulièrement préférée jusqu'à 150 mM de chlorhydrate de tris(hydroxyméthyl)aminométhane et/ou éventuellement des complexants.

21. Procédé selon la revendication 20, **caractérisé en ce que** le pH du tampon salin faible se situe dans un intervalle allant de 6 à 10 et de préférence dans un intervalle allant de 7,5 à 9,5.

22. Procédé selon la revendication 21, **caractérisé en ce qu'**une solution aqueuse de chlorhydrate de tris(hydroxyméthyl)aminométhane à 10 mM ayant un pH de 8,5 est utilisée en tant que solution saline faible.

23. Procédé selon l'une quelconque des revendications 1 à 22, **caractérisé en ce que** du sang total humain est utilisé en tant qu'échantillon biologique.

24. Procédé selon l'une quelconque des revendications 1 à 22, **caractérisé en ce qu'**une couche leucocytaire est utilisée en tant qu'échantillon biologique.

25. Procédé selon l'une quelconque des revendications 1 à 22, **caractérisé en ce qu'**une culture cellulaire est utilisée en tant qu'échantillon biologique.

26. Kit contenant des réactifs, également sous forme concentrée, pour le mélange final par l'utilisateur, contenant les solutions aqueuses suivantes :
a) du polyéthoxylate d'octylphénol, ainsi que des sels de cations monovalents, des sels de cations bivalents et un ou plusieurs complexants choisis parmi le tétraacétate d'éthylène diamine, l'acide éthylènedioxy-bis-(éthylène-nitrilo)tétraacétique, le DTPA et le Na₅P₃O₁₀, de préférence 0,5 à 200 mM de chlorure de magnésium (II) et/ou 0,5 à 200 mM de chlorure de potassium et/ou 0,5 à 100 mM de tétraacétate d'éthylène-diamine et/ou 0,5 à 100 mM d'acide éthylènedioxy-bis-(éthylène-nitrilo)tétraacétique, et de manière particulièrement préférée 1 à 30 mM de chlorure de magnésium et/ou 1 à 20 mM de chlorure de potassium et/ou 1 à 10 M de tétraacétate d'éthylène-diamine et/ou 1 à 10 mM d'acide éthylènedioxy-bis-(éthylène-nitrilo)tétraacétique, et éventuellement un système tampon à base de chlorhydrate de tris(hydroxyméthyl)aminométhane, d'acétate de sodium, de chlorhydrate de glycine, de chlorhydrate de lysine ou de chlorhydrate d'ornithine, de préférence en une concentration de 0,5 à 250 mM ;
b) une solution saline pour la resuspension du matériau contenant de l'ADN, qui contient au moins un sel chaotropique, de préférence de l'urée, de l'*iso-*thiocyanate de guanidinium ou du chlorhydrate de guanidinium ou un mélange de ces deux sels ;
c) éventuellement des enzymes pour la dégradation d'impuretés, de préférence des enzymes du groupe constitué par les protéases, les lipases, les amylases ou des mélanges d'enzymes,
d) un alcool en C₁-C₄ ramifié ou non ramifié, de préférence le propan-2-ol (*iso*-propanol) ou des mélanges de ces alcools pour la précipitation des acides nucléiques ;
e) éventuellement un liquide de lavage constitué par un mélange d'un alcanol en C₁-C₄ ramifié ou non ramifié et d'eau, de préférence un mélange d'une solution d'éthanol dans de l'eau et de manière particulièrement préférée une solution à 70 pour cent en volume d'éthanol dans de l'eau ;
f) une solution saline faible ou un solvant, dans lequel ou dans laquelle les acides nucléiques se dissolvent, ainsi qu'éventuellement des tampons, qui sont de préférence à base de tris(hydroxyméthyl)aminométhane et de manière particulièrement préférée jusqu'à 150 mM de chlorhydrate de tris(hydroxyméthyl)aminométhane, et/ou éventuellement des complexants.
